# EUROPÄISCHE PATENTSCHRIFT

(11) **EP 0 934 239 B1**
(45) Veröffentlichungstag und Bekanntmachung des Hinweises auf die Patenterteilung: **02.01.2002**
(21) Anmeldenummer: 97943884.3
(22) Anmeldetag: 23.09.1997
(51) Int. Cl.: C07C 29/17, C07C 31/20

(54) **VERFAHREN ZUR HERSTELLUNG VON 1,4-BUTANDIOL DURCH KATALYTISCHE HYDRIERUNG VON 1,4-BUTINDIOL**
PROCESS FOR PREPARING 1,4-BUTANEDIOL BY CATALYTIC HYDROGENATION OF 1,4-BUTINEDIOL
PROCEDE DE PREPARATION DE 1,4-DIOL DE BUTANE PAR HYDROGENATION CATALYTIQUE DE 1,4-DIOL DE BUTINE

(30) Priorität: 10.10.1996 DE 19641707
(43) Veröffentlichungstag der Anmeldung: 11.08.1999
(73) Patentinhaber: BASF AKTIENGESELLSCHAFT, 67056 Ludwigshafen (DE)
(72) Erfinder: BECKER, Rainer, D-67098 Bad Dürkheim (DE); BRÖCKER, Franz, Josef, D-67061 Ludwigshafen (DE); KAIBEL, Gerd, D-68623 Lampertheim (DE); PINKOS, Rolf, D-67098 Bad Dürkheim (DE); WULFF-DÖRING, Joachim, D-67227 Frankenthal (DE)
(86) Internationale Anmeldenummer: EP9705205
(87) Internationale Veröffentlichungsnummer: WO9815513

(56) Entgegenhaltungen:
- EP-A- 0 177 912
- EP-A- 0 295 435
- BE-A- 745 225

## Beschreibung

Die vorliegende Erfindung betrifft ein Verfahren zur Herstellung von 1,4-Butandiol durch katalytische Hydrierung von 1,4-Butindiol mit Wasserstoff in Gegenwart eines festen Hydrierkatalysators bei einem Druck von 1 bis 200 bar und Werten des volumenbezogenen flüssigkeitsseitigen Stoffübergangskoeffizienten k_{L}a von 0,1 s⁻¹ bis 1 s⁻¹, wobei die Flüssigkeit die kontinuierliche Phase und der Wasserstoff die disperse Phase bildet.

Die Hydrierung von 1,4-Butindiol zu 1,4-Butandiol über die im folgenden Schema vereinfacht wiedergegebenen Einzelschritte 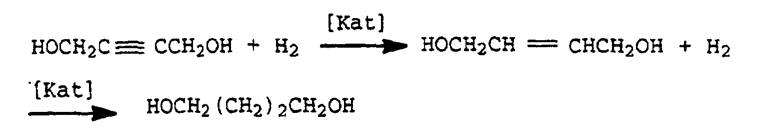 wird seit Jahrzehnten betrieben und ist vielfältig beschrieben. Die bekannten Verfahren weisen jedoch die Nachteile einer geringen unwirtschaftlichen Raum-Zeit-Ausbeute (RZA), d.h. der Menge des eingesetzten Eduktes pro Reaktorvolumen und Zeiteinheit, wenn bei Drücken unter 200 bar hydriert wird, geringer Katalysatorstandzeiten und geringer Selektivität auf. Zudem ist bei Verwendung von Festbettkatalysatoren ein investitionsintensiver hoher Druck von über 200 bar zur Durchführung der Hydrierung erforderlich.

Weiterhin sind 1,4-Butindiol, 1,4-Butendiol und daraus abgeleitete Verbindungen wie z.B. das Acetal aus Butandiol und Hydroxybutyraldehyd, das durch Isomerisierung von Butendiol gebildet wird, nicht oder nur schwer destillativ von 1,4-Butandiol zu trennen. Für die Weiterverarbeitung von 1,4-Butandiol ist es jedoch für die meisten Anwendungen kritisch, daß keine unvollständig hydrierten Produkte darin enthalten sind.

Im allgemeinen nimmt bei chemischen Reaktionen die Selektivität mit steigenden Umsätzen ab. Man ist deshalb bemüht, die Reaktion einerseits bei möglichst tiefer Temperatur, andererseits mit Teilumsatz zu betreiben, um möglichst hohe Selektivitäten zu erhalten. Bei der Butindiolhydrierung ist vollständiger Umsatz im Hinblick auf die bei der Aufarbeitung erzielbaren Produktqualität essentiell, deshalb wird die Hydrierung oft auf mehrere Reaktoren verteilt bei denen bei unterschiedlichen Bedingungen gearbeitet wird.

Aus der US-A 5 068 468 ist die Hydrierung von 1,4-Butandiol an festen geträgerten Nickel-Kupfer-Katalysatoren bekannt, bei der bei einem Druck von 250 bar Raum-Zeit-Ausbeuten von 0,3 kg Butandiol/l h erzielt werden.

In der BE-B 745 225 ist die Verwendung von Raney-Nickel-Festbettkatalysatoren bei 259 bar beschrieben, mit denen über einen zweistufigen Prozess eine Raum-Zeit-Ausbeute von 0,286 kg Butandiol/l h erzielt wird.

Aus der US-A 4 153 578 ist ein zweistufiges Verfahren zur Hydrierung von 1,4-Butindiol an suspendierten Raney-Nickel-Molybdän-Katalysatoren bei einem Druck von 21 bar bekannt. Mit diesem Verfahren werden Raum-Zeit-Ausbeuten von 0,06 kg Butandiol/l h erzielt.

Die DD-A 272 644 beschreibt die Suspensionshydrierung von wässrigem Butindiol an Nickel-SiO₂-Katalysatoren. Unter der Annahme, daß das Butindiol wie üblich 39 bis 50 gew.-%ig eingesetzt wird, errechnet sich unter der Annahme eines vollständigen Umsatzes eine Raum-Zeit-Ausbeute, die bei einem Druck von 15 bar zwischen 0,15 und 0,25 kg Butandiol/l h liegt. Der verwendete Katalysator zeigt bereits nach 50 h einen Aktivitätsverlust von 37 %.

Aus Beispiel 1 der US-A 2 967 893 läßt sich eine Raum-Zeit-Ausbeute bei der Raney-Nickel-Kupfer-katalysierten Hydrierung von 1,4-Butindiol ca. 0,01 kg Butandiol/l h errechnen.

Die RU-A 202 913 beschreibt die Hydrierung von Butindiol an einem Nickel-Chrom-Katalysator bei einer Raum-Zeit-Ausbeute von 0,1 kg Butandiol/l h.

Die EP-A 295435 beschreibt ein Verfahren zur Herstellung von Alkanolen durch katalytische Hydrierung von Alkinolen, bei dem das Alkinol vor der Hydrierung destilliert werden muß, um einen Wassergehalt von kleiner 10 Gew.-% zu erzielen. Die Hydrierung wird vorzugsweise unter Hochdruckbedingungen über 200 bar durchgeführt. Ein weiteres Hochdruckhydrierverfahren von Alkinolen ist aus EP-A 177 912 bekannt, bei dem Raum-Zeit-Ausbeuten von 0,06 kg Butandiol/l·h erzielt werden.

Aus der EP-B 0 319 208, der DE-A 19 41 633 und der DE-A 20 40 501 sind u.a. auf 1,4-Butindiol anwendbare, allgemeine Hydrierverfahren bekannt, bei denen die Kreisgasfahrweise des Reaktors durch das Durchströmen eines Festbettkatalysators von oben nach unten im Gleichstrom von Gas- und Flüssigphase vermieden werden soll. Gas- und Flüssigphase durchströmen den Katalysator dabei in Form der Übergangsströmung, wobei die Flüssigphase die kontinuierliche Phase bildet.

Diese Verfahren weisen jedoch den Nachteil auf, daß bei großen Butindiolbelastungen des Hydrierzulaufs, die Reaktionsmischung am Ende der Reaktionszone an Wasserstoff verarmt und in Folge ein nur unvollständiger Umsatz des 1,4-Butindiols erzielt wird, der zu Zwischenprodukten führt, die von Butandiol nicht oder nur schwer abtrennbar sind. Bei geringeren Butindiolbeladungen läßt sich ein vollständiger Umsatz bei ausreichender Produktqualität nur unter Inkaufnahme deutlich verringerter Raum-Zeit-Ausbeute bzw. höherem Betriebsdruck erzielen.

Der vorliegenden Erfindung lag daher die Aufgabe zugrunde, ein Verfahren zur katalytischen Hydrierung von 1,4-Butindiol zu 1,4-Butandiol zur Verfügung zu stellen, mit dem eine hohe Raum-Zeit-Ausbeute bei gleichzeitig hoher Selektivität und hohen Katalysatorstandzeiten bei einem Druck von unter 200 bar auch bei Verwendung von technischem 1,4-Butindiol erzielt werden können.

Demgemäß wurde nun ein neues und verbessertes Verfahren zur Herstellung von 1,4-Butandiol durch kontinuierliche katalytische Hydrierung von 1,4-Butindiol gefunden, dadurch gekennzeichnet, daß man 1,4-Butindiol mit Wasserstoff in der flüssigen kontinuierlichen Phase in Gegenwart eines heterogenen Hydrierkatalysators bei Temperaturen von 20 bis 300°C, bevorzugt bei 60 bis 220°C und besonders bevorzugt bei 120 bis 180°C, einem Druck von 1 bis 200 bar, bevorzugt 3 bis 150 bar und besonders bevorzugt 5 bis 100 bar, und Werten des flüssigkeitsseitigen volumenbezogenen Stoffübergangskoeffizienten k_{L}a von 0,1 s⁻¹ bis 1 s⁻¹, bevorzugt von 0,2 s⁻¹ bis 1 s⁻¹,
a) mit einem im Reaktionsmedium suspendierten Katalysator umsetzt, wobei bei Verwendung einer gepackten Blasensäule, diese in Aufwärtsfahrweise und bei einem Verhältnis von das Reaktionsgefäß verlassender zu zugeführter Gasmenge von 0,99:1 bis 0,4:1 betrieben wird, oder
b) im Gleichstrom durch einen in Kreisgasfahrweise betriebenen Festbettreaktor aufwärts hindurchleitet und dabei ein Verhältnis von das Reaktionsgefäß verlassender zu zugeführter Gasmenge von 0,99:1 bis 0,4:1 aufrecht erhält.

Mit dem erfindungsgemäßen Verfahren wird 1,4-Butandiol durch ein- oder mehrstufige Hydrierung mit hohen Raum-Zeit-Ausbeuten und bei gleichzeitig hoher Selektivität bei einem Druck unter 200 bar erhalten. Zudem können lange Katalysatorstandzeiten erzielt werden.

Der volumenbezogene flüssigkeitsseitige Stoffübergangskoeffizient zwischen der Gasphase und der Flüssigkeitsphase k_{L}a wird definiert als

k_{L}a = k_{GL} x F_{GL},

wobei k_{GL} den Stoffübergangskoeffizienten für den Stoffübergang Gas-flüssig und F_{GL} die Gas-Flüssig-Phasengrenzfläche darstellt. Der k_{L}a-Wert wird beispielsweise in Ullmanns Encyclopädie der technischen Chemie, Verlag Chemie, 4. Auflage (1973), Band 3, Seiten 495 bis 499, auch als spezifische Absorptionsgeschwindigkeit bezeichnet.

Die experimentelle Bestimmung des k_{L}a-Wertes erfolgt durch die Messung der Wasserstoffaufnahme eines Gemisches von 50 Gew.-% Butandiol und 50 Gew.-% Wasser bei der vorgesehenen Betriebstemperatur. Das Vorgehen bei der experimentellen Bestimmung des k_{L}a-Werts ist in der Fachliteratur mehrfach beschrieben, beispielsweise in P. Wilkinson et al.: "Mass Transfer and Bubble Size Distribution in a Bubble Column under Pressure", Chemical Engineering Science, Vol. 49 (1994) Nr. 9, S. 1417-1427, Ullmanns Encyclopädie der technischen Chemie, Verlag Chemie, Weinheim/Bergstr., 4. Auflage, 1973, Band 3, S. 495 - 499, H. Hoffmann: "Gepackte Aufstrom-Blasensäulen", Chem.-Ing.-Tech. 54, (1982) Nr. 10, S. 865 -876 und A. Marquez et al.: "A Review of Recent Chemical Techniques for the Determination of the Volumetric Mass-transfer Coefficient k_{L}a in Gas-liquid Reactors", Chemical Engineering and Processing, 33 (1994) S. 247 - 260.

Aufgrund der hohen k_{L}a -Werte, die bei der erfindungsgemäßen Verfahrensführung angewandt werden, ist eine Durchführung der Messung der Wasserstoffaufnahme unter kontinuierlicher Betriebsweise zu bevorzugen. Das Flüssigkeitsgemisch wird in einem möglichst großen Mengenstrom wasserstofffrei, gegebenenfalls zusammen mit suspendiertem Katalysator, mit der gewünschten Temperatur zugefahren. Der Mengenstrom des Flüssigkeitsgemisches sollte so hoch sein, daß der Flüssigkeitsinhalt im Reaktor mindestens innerhalb von 2 Minuten, bevorzugt innerhalb von 1 Minute oder weniger, ausgetauscht wird. Gleichzeitig wird wasserstoffbeladenes Flüssigkeitsgemisch entnommen, auf Normaldruck entspannt und der dabei freiwerdende gelöste Wasserstoff volumetrisch bestimmt. Der Partialdruck von Wasserstoff in der Gasphase wird ebenfalls gemessen.

Das erfindungsgemäße Verfahren wird bevorzugt mit technischem 1,4-Butindiol durchgeführt, das in Form einer wäßrigen Lösung vorliegt und als unlösliche oder gelöste Bestandteile noch Komponenten aus der Butindiolsynthese wie z.B. Kupfer-, Wismut-, Aluminium- oder Siliciumverbindungen enthalten kann. Selbstverständlich kann auch, z.B. durch Destillation gereinigtes Butindiol eingesetzt werden. Butindiol lässt sich großtechnisch aus Acetylen und wässrigem Formaldehyd herstellen und wird üblicherweise als wässrige 30 bis 60 gew.-%ige Lösung hydriert. Es kann aber auch in anderen Lösungsmitteln wie z.B. Alkoholen wie Methanol, Ethanol, Propanol, Butanol oder 1,4-Butandiol hydriert werden. Der für die Hydrierung benötigte Wasserstoff wird bevorzugt rein eingesetzt, er kann aber auch Beimengungen wie z.B. Methan und Kohlenmonoxid enthalten.

Erfindungsgemäß werden solche Katalysatoren verwendet, die C-C-Dreifach- und -Doppelbindungen zu Einfachbindungen zu hydrieren vermögen. Sie enthalten in der Regel eines oder mehrere Elemente der I., VI., VII. oder VIII.-Nebengruppe des Periodensystems der Elemente, bevorzugt die Elemente Kupfer, Chrom, Molybdän, Mangan, Rhenium, Eisen, Ruthenium, Kobalt, Nickel, Platin und Palladium. Besonders bevorzugt verwendet man Katalysatoren, die mindestens ein Element ausgewählt aus Kupfer, Chrom, Molybdän, Eisen, Nickel, Platin und Palladium enthalten.

Der Metallgehalt dieser Katalysatoren liegt in der Regel zwischen 0,1 - 100 Gew.-%, bevorzugt 0,2 - 95 Gew.-%, besonders bevorzugt 0,5 - 95 Gew.-%.

Der Katalysators enthält bevorzugt zusätzlich mindestens ein Element ausgewählt aus den Elementen der II. ,III.,IV. und VI.-Hauptgruppe, der II., III., IV. und V. Nebengruppe des Periodensystems der Elemente und der Lanthanoiden als Promotor zur Aktivitätssteigerung.

Der Promotorgehalt des Katalysators beträgt in der Regel bis 5 Gew.-%, bevorzugt 0,001 - 5 Gew.-%, besonders bevorzugt 0,01 - 3 Gew.-%.

Als Katalysatoren können Fällungs-, Träger-, oder Raney-Typ-Katalysatoren verwendet werden, deren Herstellung beispielsweise in Ullmanns, Encyclopädie der technischen Chemie, 4. Auflage, 1977, Band 13, Seiten 558-665 beschrieben ist.

Als Trägermaterialien können Aluminiumoxide, Titanoxide, Zirkoniumdioxid, Siliciumdioxid, Tonerden, z.B. Montmorillonite, Silikate wie Magnesium- oder Aluminiumsilikate, Zeolithe sowie Aktivkohlen verwendet werden. Bevorzugte Trägermaterialien sind Aluminiumoxide, Titandioxide, Siliciumdioxid, Zirkoniumdioxid und Aktivkohlen. Selbstverständlich können auch Mischungen verschiedener Trägermaterialien als Träger für im erfindungsgemäßen Verfahren anwendbare Katalysatoren dienen.

Diese Katalysatoren können entweder als Katalysatorformkörper, beispielsweise als Kugeln, Zylinder, Ringe, Spiralen, oder in Form von Pulvern verwendet werden.

Als Raney-Typ-Katalysatoren sind beispielsweise Raney-Nickel, Raney-Kupfer, Raney-Kobalt, Raney-Nickel/Molybdän, Raney-Nickel/Kupfer, Raney-Nickel/Chrom, Raney-Nickel/Chrom/Eisen oder Rhenium-Schwamm geeignet. Raney-Nickel/Molybdän-Katalysatoren können beispielsweise nach dem in der US-A 4 153 578 beschriebenen Verfahren hergestellt werden. Diese Katalysatoren werden aber auch beispielsweise von der Firma Degussa 63403 Hanau, Deutschland vertrieben. Ein Raney-Nickel-Chrom-Eisen-Katalysator wird beispielsweise unter der Handelsbezeichnung Katalysator Typ 11 112 W® von der Firma Degussa vertrieben.

Bei der Verwendung von Fäll- oder Trägerkatalysatoren werden diese zu Beginn der Reaktion bei 150 und 500°C im Wasserstoff-bzw. Wasserstoff/Inertgas-Strom reduziert. Diese Reduktion kann direkt im Synthese-Reaktor durchgeführt werden. Falls die Reduktion in einem separaten Reaktor durchgeführt wird, können die Katalysatoren vor dem Ausbau bei 30°C mit sauerstoffhaltigen Gasgemischen oberflächlich passiviert werden. Die passivierten Katalysatoren können in diesem Fall im Synthese-Reaktor vor dem Einsatz in einem Stickstoff/Wasserstoffstrom bei 180°C aktiviert oder auch ohne Aktivierung eingesetzt werden.

Die Katalysatoren können im Festbett oder in Suspension eingesetzt werden. Wenn die Katalysatoren in Form eines Festbetts angeordnet sind, wird der Reaktor erfindungsgemäß nicht in der üblichen Rieselfahrweise, sondern in einem aufwärts gerichteten Gleichstrom von Flüssigkeit und Gas so betrieben, daß die Flüssigkeit und nicht das Gas als kontinuierliche Phase vorliegt.

Suspendierte Katalysatoren werden mit einer Korngröße im allgemeinen von 0,1 - 500 µm, bevorzugt 0,5 bis 200 µm, besonders bevorzugt 1 bis 100 µm eingesetzt.

Wird mit suspendierten Katalysatoren gearbeitet, so wird bei Verwendung von gepackten Blasensäulen ebenfalls mit einem aufwärts gerichteten Gleichstrom von Flüssigkeit und Gas so gearbeitet, daß die Flüssigkeit und nicht das Gas als kontinuierliche Phase vorliegt. Das Verhältnis von das Reaktionsgefäß verlassender zu zugeführter Gasmenge beträgt bei der Verwendung von Festbettreaktoren und bei der Verwendung von gepackten Blasensäulen mit einem im Reaktionsmedium suspendierten Katalysator 0,99:1 bis 0,4:1.

Das erfindungsgemäß bei Festbettreaktoren und bei im Reaktionsmedium suspendierten Katalysatoren in gepackten Blasensäulen einzuhaltende Verhältnis von das Reaktionsgefäss verlassender zu zugeführter Gasmenge lässt sich in einfacher Weise einstellen, indem man die entsprechende Menge Wasserstoff entweder als Frischgas dosiert, oder technisch bevorzugt, Kreisgas rückführt und nur den durch chemischen Verbrauch und Abgas bedingten Wasserstoffverlust durch Frischwasserstoff ergänzt.

Das molare Verhältnis von Wasserstoff zu Butindiol im Reaktor beträgt mindestens 3:1,bevorzugt zwischen 4:1 und 100:1.

Das erfindungsgemäße Verfahren wird an Festbettkatalysatoren in Kreisgasfahrweise durchgeführt, d.h. das den Reaktor verlassende Gas wird im Kreislauf, gegebenenfalls nach Ergänzung mit frischem Wasserstoff, über einen Verdichter in den Reaktor rückgeführt. Es ist möglich, die gesamte Kreisgasmenge oder eine Teilmenge davon über einen Treibstrahlverdichter zu führen. In dieser bevorzugten Ausführungsform wird der Kreisgasverdichter durch eine kostengünstige Düse ersetzt. Die Verdichtungsarbeit wird über die ebenfalls im Kreis geführte Flüssigkeit eingebracht. Die erforderliche Druckerhöhung der Flüssigkeit zum Betreiben des Treibstrahlverdichters beträgt etwa 3 bis 5 bar.

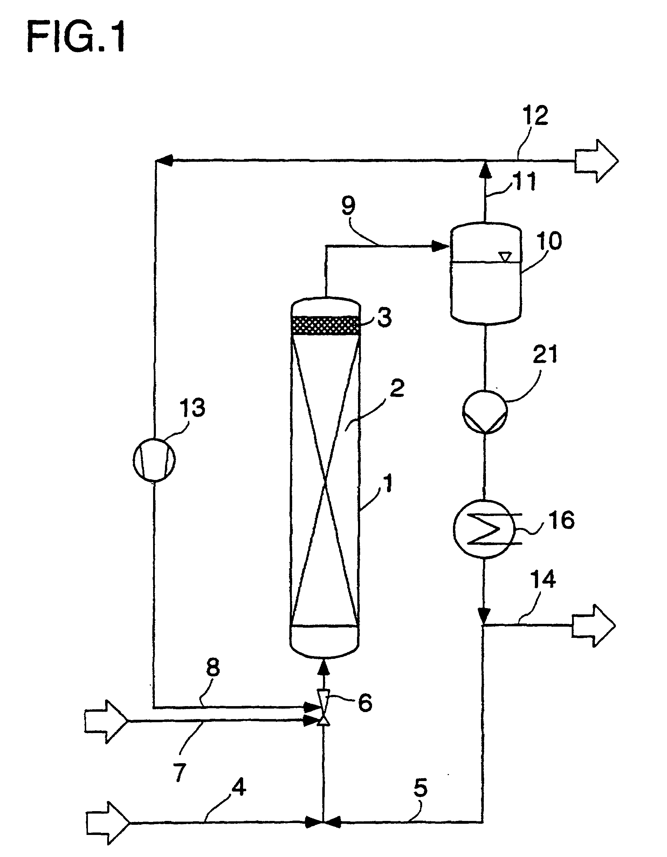

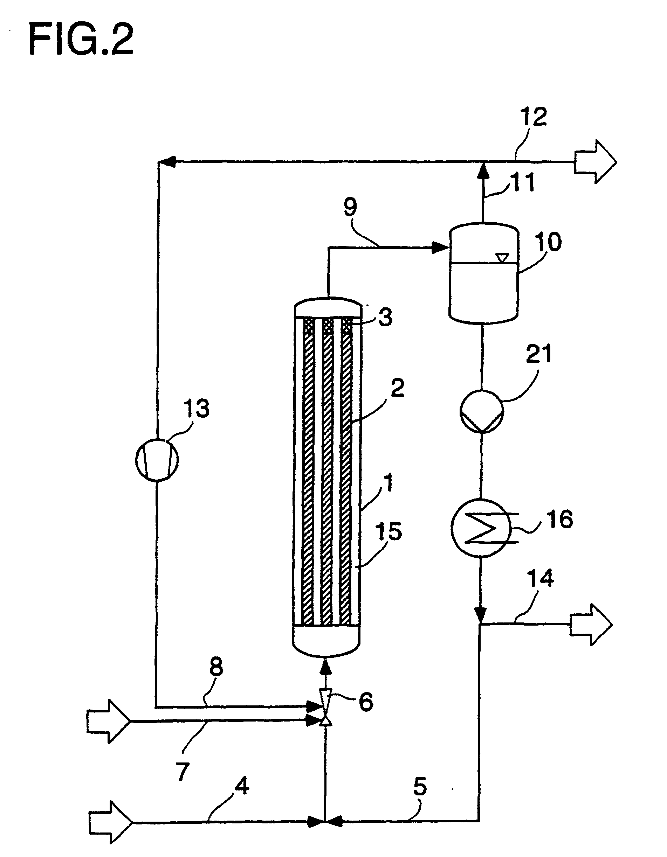

Für die Durchführung des erfindungsgemäßen Verfahrens an Katalysatoren im Festbett geeignete Reaktoren sind beispielsweise der Festbettreaktor gemäß Abb. 1 oder ein Rohrbündelreaktor gemäß Abb. 2.

Abb. 1 zeigt schematisch die Anordnung eines im erfindungsgemäßen Verfahren verwendbaren Festbettreaktors. In dem Reaktorbehälter 1 befindet sich eine Schüttung von Katalysatorteilchen 2 mit einem mittleren Durchmesser von etwa 1 bis 20 mm, bevorzugt 2 bis 5 mm. Zur Verhinderung des Austrags von Katalysatorteilchen befindet sich am oberen Ende der Katalysatorschüttung ein Drahtgestrick 3. Der aus Butindiol und Wasser bestehende flüssige Zulauf 4 wird vorteilhaft über die Leitung zusammen mit Kreislaufflüssigkeit über Leitung 5 als Treibstrahl in eine Mischdüse 6 gefahren, in der Frischwasserstoff über Leitung 7 und Kreisgas über Leitung 8 zugemischt werden. Am oberen Ende des Reaktors 1 tritt ein zweiphasiges Gas/Flüssigkeits-Gemisch 9 aus, das in einem Gasflüssigkeitsabscheider 10 getrennt wird. Aus dem Gasstrom 11 wird zur Vermeidung der Aufpegelung von Inertbestandteilen ein Teilstrom 12 entnommen und ausgeschleust. Der Kreisgasstrom 8 wird über einen Verdichter 13 in die Mischdüse 6 zurückgeführt. Dieser Verdichter kann gegebenenfalls entfallen, wenn die Kreislaufflüssigkeit 5, die über die Pumpe 21 geführt wird, mit ausreichend hohem Druck bereitgestellt werden kann und die Mischdüse 6 als Treibstrahlverdichter ausgeführt wird. Aus der Kreislaufflüssigkeit wird ein Teilstrom 14 als Produktstrom entnommen. Die freiwerdende Reaktionswärme wird im Wärmetauscher 16 abgeführt.

Das erfindungsgemäße Verfahren kann außer in dem in Abb. 1 beschriebenen adiabatisch betriebenen Festbettreaktor auch in dem in Abb. 2 beschriebenen isotherm betriebenen Rohrbündelrekator durchgeführt werden.

Abb. 2 zeigt schematisch die Anordnung eines Rohrbündelreaktors, bei dem die Katalysatorteilchen 2 mit einem mittleren Durchmesser von etwa 1 bis 20 mm, bevorzugt 2 bis 5 mm in den Rohren 15 angeordnet sind.

Das Mengenverhältnis von Kreislaufflüssigkeit 5 zum Austrag 14 beträgt sowohl beim Festbettreaktor gemäß Abb. 1 als auch beim Rohrbündelreaktor gemäß Abb. 2 100:1 bis 500:1, bevorzugt 200:1. Der Reaktor ist hinsichtlich seines Durchmessers so dimensioniert, daß sich für die Flüssigkeit eine Leerrohrgeschwindigkeit von 100 bis 1.000 m/h einstellt. Die geeignete Leerrohrgeschwindigkeit wird für jeden Katalysatortyp in einer Laborapparatur ermittelt. Es empfiehlt sich, als Leerrohrgeschwindigkeit die im Hinblick auf den Katalysatorabrieb maximal zulässige Geschwindigkeit einzustellen. Bei Leerrohrgeschwindigkeiten oberhalb von etwa 1000m/h zeigte sich, daß bei kleinen Katalysatorteilchen eine zusätzliche Begrenzung durch den zunehmenden Druckverlust wirksam wird.

Haupteinflussgrössen für die Festlegung der Leerrohrgeschwindigkeit sind die Katalysatorabmessungen, seine Form und Korngrössenverteilung und sein Abriebverhalten. Als Anhaltswert kann erfahrungsgemäß gelten, daß der Druckverlust etwa 0,02 bis 0,15 bar/m beträgt. Die Menge des Gases am Reaktoraustrag wird bevorzugt so eingestellt werden, daß sich eine Gasleerrohrgeschwindigkeit ergibt, die der Flüssigkeitsleerrohrgeschwindigkeit etwa vergleichbar ist. Sie kann jedoch gegebenenfalls um bis zu 90 % niedriger liegen.

Für die Durchführung des erfindungsgemäßen Verfahrens mit einem im Reaktionsmedium suspendierten Katalysator sind Strahldüsenreaktoren, Rührkessel und Blasensäulen mit Packungen, welche eine Packungsoberfläche von mindestens 500, bevorzugt 1000 bis 2000 m²/m³ aufweisen, geeignet. Strahldüsenreaktoren können in verschiedenen Bauarten Anwendung finden, wenn sie durch einen ausreichend hohen Energieeintrag, der erfahrungsgemäß oberhalb von 2 kW/m³ liegt, den für die Erfindung wesentlichen hohen Stoffübergang von der Gasphase an die Flüssigkeit mit den suspendierten Katalysatorteilchen gewährleisten können. Besonders geeignet sind Strahldüsenreaktoren, die mit einem Impulsaustauschrohr ausgerüstet sind. Eine industriell verbreitete Bauform eines Strahldüsenreaktors ist beispielsweise der in der EP-A 0 419 419 beschriebene Reaktor. Bei Werten für den Energieeintrag von 3 bis 5 kW/m³ ist mit diesem Reaktor eine Abscheidung der Gasphase noch in einfachen Abscheidern möglich, ohne Zusatzapparate wie Schaumzentrifugen oder Zyklone, benutzen zu müssen.

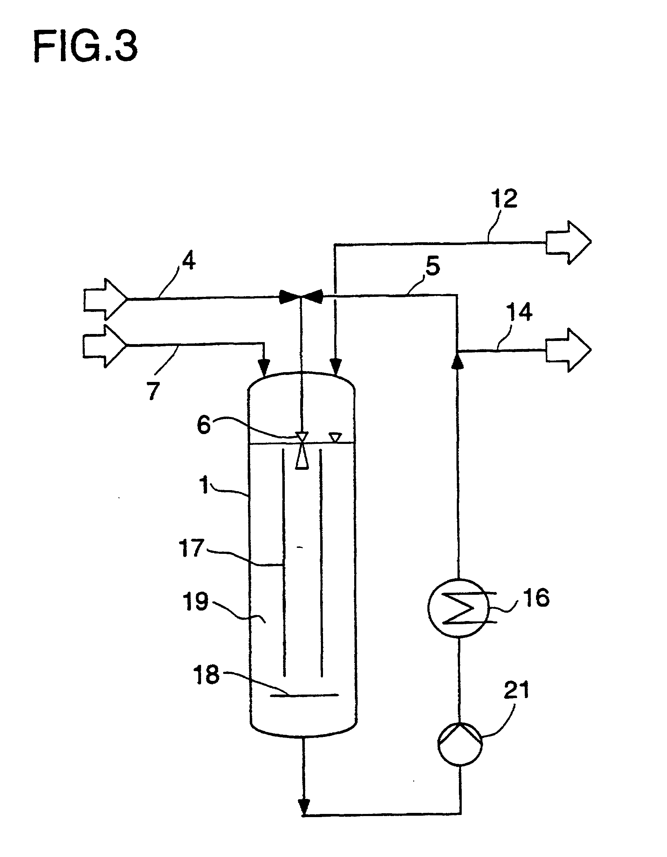

In Abb. 3 ist ein Strahldüsenreaktor gezeigt, bei dem die Flüssigkeit über Leitung 5 über einen äußeren Kreislauf mit Wärmetauscher 16 geführt wird und in einem Treibstrahlverdichter 6 Wasserstoff ansaugt. Zur Intensivierung des Stoffaustausches wird das zweiphasige Gemisch über ein Impulsaustauschrohr 17 geführt. Am unteren Ende des Reaktors 1 befindet sich gegebenenfalls eine Prallplatte 18, die die Strömung umlenkt und die Abscheidung des Gases erleichtert. Das Gas steigt im äusseren Ringraum 19 nach oben und wird von dem Treibstrahlverdichter 6 erneut angesaugt. Die Flüssigkeit, aus der das Gas im wesentlichen abgeschieden ist, wird am unteren Ende des Reaktors entnommen, über einen Wärmetauscher 16 zur Abführung der Reaktionswärme geführt und erneut in den Treibstrahlverdichter 6 gegeben.

Rührbehälter sind für die Durchführung des erfindungsgemäßen Verfahrens nur dann geeignet, wenn der Energieeintrag in einem Bereich von 2 bis 10 kW/m³ liegt. Um die Rührerenergie so umzusetzen, daß ein erfindungsgemäß hoher k_{L}a-Wert erreicht wird, ist es sinnvoll, Einbauten im Rührbehälter zu haben, die die innige Durchmischung von Gas- und Flüssigkeit gewährleisten, wie zum Beispiel Stromstörer, die auch als Wellenbrecher bezeichnet werden.

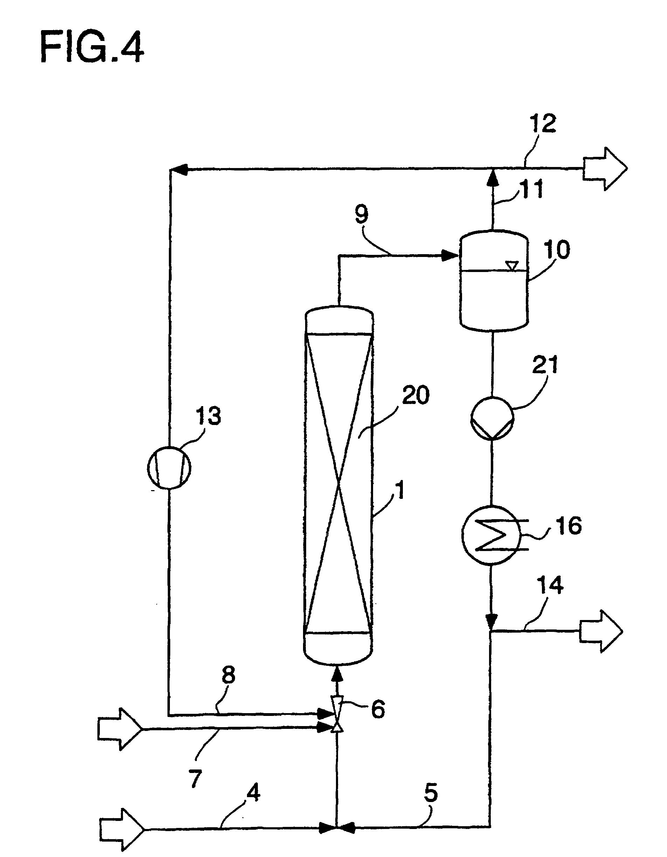

Weiterhin sind die in Abb. 4 gezeigten mit Packungen 20 ausgerüsteten Blasensäulen für das erfindungsgemäße Verfahren geeignet. Die Packungsoberfläche muß mindestens 500 m²/m³, vorzugsweise 1000 bis 2000 m²/m³ betragen. Die Packungen 20 können geordnet oder ungeordnet sein, wobei geordnete Pakkungen, wie sie hinsichtlich ihrer Geometrie von der destillativen Trenntechnik bekannt sind, den niedrigsten Druckverlust aufweisen. Besonders günstige Eigenschaften zeigten Pakkungen aus Maschendrahtgeweben, wie sie in ähnlicher Form in der Destillationstechnik eingesetzt werden, beispielsweise die Gewebepackungen Sulzer DX® oder Sulzer EX® , die von der Firma Sulzer Chemtechn., 8404 Winterthur, Schweiz, vertrieben werden.

Die genannten Packungen lassen sich auch direkt mit katalytisch aktiven Komponenten beschichten. Derartige Packungen sind in der EP-A 068 862, der EP-A 201 614 und der EP-A 448 884 beschrieben. Der durch Verwendung einer mit diesen Packungen gepackten Blasensäule erhaltene Festbettreaktor weist bei gleich hoch eingestellten Leerrohrgeschwindigkeiten für die Flüssigkeit und das Gas von jeweils 100 bis 1000 m/h, bevorzugt 200 bis 1000 m/h, die gleichen hohen k_{L}a-Werte auf wie bei der Suspensionsfahrweise.

Die Einstellung der erfindungsgemäßen, für das gleichzeitige Erreichen einer hohen Selektivität und einer hohen Raum-Zeit-Ausbeute entscheidenden k_{L}a-Werte von 0,1 s⁻¹ bis 1 s⁻¹ erfolgt durch auf den jeweiligen Reaktortyp zugeschnittene gezielte technische Maßnahmen. Für alle Reaktortypen gemeinsam ist ein im Vergleich zu anderen Verfahrensführungen erhöhter Energieeintrag. Durch spezielle konstruktive Ausgestaltungen und Fahrbedingungen wird die eingebrachte Energie möglichst wirksam für die Verbesserung des Stoffübergangs umgesetzt.

Bei Verwendung von suspendierten Katalysatoren in Rührbehältern müssen zur Einstellung der k_{L}a-Werte von 0,1 s⁻¹ bis 1 s⁻¹ in dem erfindungsgemäßen Verfahren Rührertypen mit guten Begasungseigenschaften, beispielsweise Scheibenrührer oder Schrägblattrührer, wie sie u.a. aus der Fermentationstechnik bekannt sind, eingesetzt werden. Der volumenbezogene Energieeintrag liegt zwischen 2 und 10 kW/m³, wobei der untere Wert nur für kleine Apparategrößen gute Hydrierergebnisse erlaubt. Bei Reaktorgrößen oberhalb von etwa 0,5 m³ sind Energieeinträge von 5 - 10 kW/m³ zu erforderlich. Der Energieeintrag erfolgt bei Rührbehältern über die Antriebsleistung des Rührers. Diese Werte des Energieeintrags liegen höher als bei üblichen Begasungsreaktionen in Rührbehältern, wie beispielsweise Fermentationen oder Hydrierungen, bei denen der Energieeintrag zwischen etwa 0,2 und 1,0 kW/m³ liegt.

Strahldüsenreaktoren mit suspendierten Katalysatoren benötigen volumenbezogene Energieeinträge von mehr als 2 kW/m³, bevorzugt 3 - 5 kW/m³. Der Energieeintrag erfolgt durch die Druckerhöhung der Flüssigkeit in der Umwälzpumpe 21 in Verbindung mit dem Druckabbau im Treibstrahlverdichter 6. Durch Variation von Umwälzmenge und Druckaufbau in der Umwälzpumpe läßt sich der gewünschte Energieeintrag einstellen. Der Druckaufbau in der Pumpe liegt üblicherweise im Bereich von 2 bis 5 bar.

Werden im Reaktionsmedium suspendierte Katalysatoren in gepackten Blasensäulen in dem erfindungsgemäßen Verfahren verwendet, so muß die volumenbezogene Oberfläche der Packungen mindestens 500 m²/m³, bevorzugt jedoch 1000 - 2000 m²/m³ betragen. Zur Einstellung der erfindungsgemäßen k_{L}a-Werte von 0,1 s⁻¹ bis 1 s⁻¹ sind gleichzeitig hohe Geschwindigkeiten für die Flüssigkeit und das Gas von 100 und 1000 m/h erforderlich. Die Einstellung der angegebenen Geschwindigkeiten von Flüssigkeit und Gas gewährleistet in Verbindung mit den angegebenen Geometrien der Packungen die Einstellung des erforderlichen Energieeintrags, der bauartbedingt jedoch niedriger liegt als bei Rührbehältern oder Strahldüsenreaktoren. Das Erreichen des geeigneten Energieeintrags, der durch den Druckverlust der strömenden Flüssigkeit und des Gases entsteht, kann durch Messung des resultierenden Druckverlustes, der zwischen 0,02 und 0,15 bar/m Packung liegt, kontrolliert werden. Gegebenenfalls kann die Geschwindigkeit der Flüssigkeit entsprechend geändert werden, um den gewünschten Druckabfall einzustellen.

Die vorstehend für die Suspensionsfahrweise in gepackten Blasensäulen angegebenen Werte für die volumenbezogene Oberfläche, den Druckabfall in der Packung sowie und die Umwälzgeschwindigkeiten für die Flüssigkeit und das Gas gelten auch für gepackte Blasensäulen, bei denen die Packungen selbst mit katalytisch aktivem Material beschichtet sind.

Bei Verwendung von Festbettreaktoren in dem erfindungsgemäßen Verfahren muß zur Einstellung von k_{L}a-Werte von 0,1 s⁻¹ bis 1 s⁻¹ die mittlere Größe der Katalysatorteilchen 1 - 20 mm, bevorzugt 2 - 5 mm und die Geschwindigkeit der durchströmenden Flüssigkeit und des Gases 100 - 1000 m/h betragen. Der sich einstellende Druckverlust soll etwa 0,02 - 0,15 bar/m Festbett betragen.

1,4-Butandiol findet in der Technik in großen Mengen Anwendung z.B. bei der THF-Herstellung oder als Diolkomponente in Polyestern.

Das erfindungsgemäße Verfahren wird anhand der nachfolgenden Beispiele näher erläutert. Wenn nicht anders angegeben, wurde technisches Butindiol in Form einer 54 gew. -%igen wässrigen Lösung eingesetzt, welche wechselnde Mengen Propinol enthielt. Die Mengen an Propinol entsprechen in etwa den in den Beispielen angegebenen Mengen Propanol im Reaktionsaustrag. Die Prozentangaben der Reaktionsausträge in den Beispielen stellen, wenn nicht anders vermerkt, wasserfrei gerechnete Gewichtsprozente dar, die gaschromatographisch ermittelt worden sind.

### Beispiele

### Beispiel 1

In einen Rührautoklaven mit einem Flüssigkeitsstand von 130 ml mit zwei Wellenbrechern, Scheibenrührern und eingebauter Standhaltung (einer Metallsinterfritte zum Zurückhalten des Katalysators) wurden 10 g Raney-Ni/Mo (2 Gew.-% Molybdän-Gehalt, berechnet als Mo, durch Tränken des Raney-Nickels mit Ammoniummolybdat-Lösung) in 50 ml Wasser gefüllt und mit Wasserstoff anschliessend auf 35 bar gebracht. Mittels einer aussenliegenden Ölheizung wurde dann die Reaktorinnentemperatur auf 140°C gebracht und ein Wasserstoffstrom von 80 Normlitern/h eingestellt. Der Rührer wurde auf 700 U/min eingestellt, was einen k_{L}a-Wert von 0,2 s⁻¹ gewährleistete. Sodann wurden 100 g/h einer 54 gew.-%igen wässrigen Butindiol-Lösung zugepumpt. Die Reaktorinnentemperatur stieg auf 149°C. Im Reaktionsaustrag von 103 g/h fanden sich 94,2 Gew.-% 1,4-Butandiol, 1,3 Gew.-% n-Butanol, 3,3 Gew.-% n-Propanol sowie einige weitere, mengenmässig jeweils unter 0,08 Gew.-% liegende Produkte. Die RZA betrug 0,4 kg Butandiol/l h.

### Beispiel 2

Analog Beispiel 1 wurden 170 g/h Butindiol-Lösung an 10 g Raney-Ni/Mo (2,5 Gew.-% Molybdän-Gehalt, berechnet als Mo,) hydriert. Die Ausgangstemperatur betrug 150°C, während der Reaktion stieg die Reaktortemperatur auf 173°C. Im Austrag von 176 g/h fanden sich 92,4 Gew.-% 1,4-Butandiol, 0,4 Gew.-% 2-Methylbutandiol, 2 Gew.-% n-Butanol und 4,7 Gew.-% n-Propanol sowie einige weitere, mengenmäßig unter 0,08 Gew.-% liegende Produkte. Die RZA betrug 0,7 kg Butandiol/l h.

### Beispiel 3

Analog Beispiel 1 wurden 60 g Butindiol-Lösung an 10 g Raney-Ni/Fe/Cr (Typ 11 112 W von Degussa) hydriert. Der Flüssigkeitsstand im Reaktor betrug 85 ml, der k_{L}a-Wert betrug 0,2 s⁻¹. Die Ausgangstemperatur lag bei 140°C und stieg dann auf 144°C an. Im Austrag von 64 g/h fanden sich 95,7 Gew.-% Butandiol, 0,6 Gew.-% n-Butanol und 1,8 Gew.-% n-Propanol sowie einige weitere, mengenmässig unter 0,08 Gew.-% liegende Produkte. Die RZA betrug 0,25 kg Butandiol/l h.

### Beispiel 4

Analog Beispiel 1 wurden bei 600 U/Min., entsprechend einem k_{L}a-Wert von 0,1 s⁻¹, und einer Reaktortemperatur von 105°C 60 g/h Butindiollösung hydriert. Im Reaktoraustrag fanden sich 90 Gew.-% Butandiol, 1,8 Gew.-% Butendiol, 5 Gew.-% des Acetals aus 4-Hydroxybutyraldehyd und Butandiol, 2 Gew.-% 4-Hydroxybutyraldehyd und 4 Gew.-% Butanol. Nach Erhöhung der Reaktortemperatur auf 136°C stieg der Umsatz und es wurden im Austrag folgende Gehalte der Produkte bestimmt: 92 Gew.-%, Butandiol, 2,7 Gew.-% Acetal aus 4-Hydroxybutyraldehyd und Butandiol, 0,7 Gew.-% 4-Hydroxybutyr-aldehyd und 3 Gew.-%. Butanol. Beispiel 4 zeigt, daß durch das erfindungsgemäße Verfahren bei höheren Umsätzen höhere Selektivitäten erreichbar sind.

### Beispiel 5

Ein 400 ml ölbeheizter Rohrreaktor mit 2,7 cm Durchmesser wurde mit 400 ml Raschigringen aus Metallgewebsringen mit den Werkstoffnummern 1.4541, Stahleisenliste, herausgegeben vom Verein Deutscher Eisenhüttenleute, 8. Aufl., Verlag Stahleisen mbH, Düsseldorf 1990, (UNS-No. S 32100) mit 5 mm Durchmesser gefüllt. Der Rohrreaktor wurde in ein Reaktionssystem eingebaut, bei dem über einen Gas/Flüssigabscheider mittels einer Zahnradpumpe Reaktionsflüssigkeit umgepumpt werden konnte. Im Abscheider befand sich ein Filter, durch den Flüssigkeit und Gas kontinuierlich abgezogen werden konnten, der Katalysator aber zurückgehalten wurde. Der Zulauf von 200 g Butindiol analog Beispiel 1 und 100 Nl/h Frischgas wurde vor dem Reaktor zugefahren. Der Reaktor wurde in Sumpffahrweise betrieben. Die Flüssigkeitsbelastung betrug 170 m³/m²h, der k_{L}a-Wert 0,25 s⁻¹.

Vor der Reaktion wurden analog. Beispiel 1 20 g Raney-Ni/Mo in 300 ml Wasser ins Reaktorsystem eingefüllt. Bei 30 bar und 145 bis 151°C Reaktortemperatur wurden im Austrag von 213 g/h 93,3 Gew.-% Butandiol, 0,3 Gew.-% 2-Methylbutandiol, 1,5 Gew.-% n-Butanol, 4,2 Gew.-% n-Propanol sowie einige weitere, mengenmässig unter 0,08 Gew.-% liegende Produkte gefunden. Die RZA betrug ca. 0,25 kg Butandiol/l h.

### Beispiel 6

Analog Beispiel 5 wurden 5 g Raney-Ni/Mo eingebaut. Bei ca. 122°C Reaktortemperatur, 20 bar und 300 l/h Wasserstoff wurden 100 g Butindiollösung hydriert. Bei einer Flüssigkeitsbelastung von 225 m³/m²h und einem k_{L}a-Wert 0,3 s⁻¹ wurden bei vollständigem Butindiol-Umsatz n-Butanol-Gehalte im Austrag von 2,2 - 2,7 Gew.-% erhalten, wobei der 1,4-Butandiol-Gehalt bei 77 Gew.-% lag, der Rest waren Zwischenprodukte. Nach Steigerung der Flüssigkeitsbelastung auf 263 m³/m²h, entsprechend einem k_{L}a -Wert von 0,4 s⁻¹, sank der n-Butanol-Gehalt auf 1,3 Gew.-% ab, der Butandiol-Gehalt stieg auf 88 Gew.-%.

### Beispiel 7

Analog Beispiel 1 wurden bei 127°C Reaktortemperatur und einem k_{L}a-Wert von 0,2 s⁻¹ 60 g/h 57 gew.-%ige wässrige Butindiollösung { Wassergehalt : 42 Gew.-%) hydriert. Nach 125 h Reaktionszeit fanden sich im Austrag 95,4 Gew.-% Butandiol, 0,1 Gew.-% 2-Methylbutandiol, 1,5 Gew.-% Acetal aus 4-Hydoxybutyraldehyd und Butandiol, 2,6 Gew.-% Butanol und 0,3 Gew.-% Propanol. Danach wurde die Reaktortemperatur auf 141°C erhöht. In der Folgezeit vervollständigte sich der Umsatz auch der Zwischenprodukte und die Selektivität stieg. Nach 173 h Betriebszeit wurden folgende Gehalte bestimmt: 98,8 Gew.-% Butandiol, 0,1 Gew.-% 2-Methyl-butandiol, 0,7 Gew.-% Butanol und 0,3 Gew.-% Propanol.

### Vergleichsbeispiel 1:

Analog Beispiel 1 wurde destilliertes Butindiol als 50 gew.-%ige wässrige Lösung hydriert. Der pH der Zulauflösung wurde mittels NaOH auf 6,6 eingestellt. Bei 140°C Ölbadtemperatur stellte sich bei 100 g/h Zulauf eine Reaktorinnentemperatur von 150°C ein. Im Reaktoraustrag fanden sich nach 24 h Betriebszeit 3 Gew.-% n-Butanol, 0,5 Gew.-% n-Propanol und 96 Gew.-% 1,4-Butandiol. Nach Zurücknahme der Rührerdrehzahl auf 350 U/Min., entsprechend einem k_{L}a-Wert von 0,05 s⁻¹, sank die Reaktortemperatur auf 141°C und es fanden sich im Reaktoraustrag 10 Gew.-% Butindiol, 31 Gew.-% Butendiol, 41 Gew.-% Butandiol, 3 Gew.-% 4-Hydroxy-butyraldehyd, 0,5 Gew.-% Propanol sowie 4 Gew.-% Butanol und 7 Gew.-% Butenole. Den verbleibende Rest bildeten überwiegend Acetale.

### Vergleichsbeispiel: 2

Analog Beispiel 1 wurden mit 10 g Ra-Ni/Mo (1,8 Gew.-% Molybdän, berechnet als Mo) 100 g/h technische Butindiollösung bei einer Ölbadtemperatur von 140°C, umgesetzt. Die Reaktorinnentemperatur lag dabei bei 149°C. Der Reaktionsaustrag hatte folgende Zusammensetzung: 94,1 Gew.-% Butandiol, 0,2 Gew.-% 2-Methylbutandiol, 1,5 Gew.-% Butanol und 4,2 Gew.-% Propanol. Nach Verminderung der Rührerdrehzahl auf 350 U/Min., entsprechend einem k_{L}a-Wert von 0,05 s⁻¹ wurde folgendes Hydrierergebnis erhalten: 40,3 Gew.-% Butandiol, 37 Gew.-% Butendiol, 2,1 Gew.-% Butindiol, 3 Gew.-% Butanol, 2,6 Gew.-% Butenole, 3,1 Gew.-% 4-Hydroxybutyraldehyd.

Der zu 100 Gew.-% fehlende Rest bestand überwiegend aus Propanol, Propenol und Acetalen aus 4-Hydroxybutyraldehyd mit den Diolen.

## Patentansprüche

1. Verfahren zur Herstellung von 1,4-Butandiol durch kontinuierliche katalytische Hydrierung von 1,4-Butindiol, **dadurch gekennzeichnet, daß** man 1,4-Butindiol mit Wasserstoff in der flüssigen kontinuierlichen Phase in Gegenwart eines heterogenen Hydrierkatalysators bei Temperaturen von 20 bis 300°C, einem Druck von 1 bis 200 bar und Werten des flüssigkeitsseitigen volumenbezogenen Stoffübergangskoeffizienten k_{L}a von 0,1s⁻¹ bis 1 s⁻¹.
a) mit einem im Reaktionsmedium suspendierten Katalysator umsetzt, wobei bei Verwendung einer gepackten Blasensäule, diese in Aufwärtsfahrweise und bei einem Verhältnis von das Reaktionsgefäß verlassender zu zugeführter Gasmenge von 0,99:1 bis 0,4:1 betrieben wird, oder
b) im Gleichstrom durch einen in Kreisgasfahrweise betriebenen Festbettreaktor aufwärts hindurchleitet und dabei ein Verhältnis von das Reaktionsgefäß verlassender zu zugeführter Gasmenge von 0,99:1 bis 0,4:1 aufrecht erhält.

2. Verfahren nach Anspruch 1, **dadurch gekennzeichnet, daß** es bei einem Druck von 3 bis 150 bar durchgeführt wird.

3. Verfahren nach Anspruch 1, **dadurch gekennzeichnet, daß** es bei einem Druck von 5 bis 100 bar durchgeführt wird.

4. Verfahren nach einem der Ansprüche 1 bis 3, **dadurch gekennzeichnet, daß** der flüssigkeitsseitige volumenbezogene Stoffübergangskoeffizient 0,2 s⁻¹ bis 1 s⁻¹ beträgt.

5. Verfahren nach einem der Ansprüche 1 bis 4, **dadurch gekennzeichnet, daß** man einen Katalysator verwendet, der mindestens ein Element ausgewählt aus den Elementen der I., VI., VII. oder VIII. -Nebengruppe des Periodensystems der Elemente enthält.

6. Verfahren nach Anspruch 5, **dadurch gekennzeichnet, daß** der Katalysator mindestens eines der Elemente Kupfer, Chrom, Molybdän, Mangan, Rhenium, Eisen, Ruthenium, Kobalt, Nickel, Platin und Palladium enthält.

7. Verfahren nach einem der Ansprüche 5 oder 6, **dadurch gekennzeichnet, daß** der Katalysator bis zu 5 Gew. -% mindestens eines Elementes ausgewählt aus den Elementen der II., III., IV. und VI. -Hauptgruppe, der II., III., IV. und V.-Nebengruppe des Periodensystems der Elemente und den Lanthanoiden enthält.

8. Verfahren nach einem der Ansprüche 5 bis 7, **dadurch gekennzeichnet, daß** der Katalysator einen Träger ausgewählt aus den Oxiden des Aluminiums und Titans, Zirkoniumdioxid, Siliciumdioxid, Tonerden, Silikaten, Zeolithen und Aktivkohle aufweist.

## Claims

1. A process for preparing 1,4-butanediol by continuous catalytic hydrogenation of 1,4-butynediol, **characterized in that** 1,4-butynediol with hydrogen is in the liquid continuous phase in the presence of a hydrogenation catalyst at from 20 to 300°C, a pressure of from 1 to 200 bar and values of the liquid-side volumetric mass transfer coefficient k_{L}a of from 0.1 s⁻¹ to 1 s⁻¹
a) reacted with a catalyst suspended in the reaction medium, where if a packed bubble column is employed this is operated in the upflow mode and at a ratio of gas leaving the reaction vessel to gas fed to the reaction vessel of from 0.99:1 to 0.4:1, or
b) passed in cocurrent in an upward direction through a fixed-bed reactor operated in the gas-circulation mode while maintaining a ratio of the gas fed to the reaction vessel to gas leaving the reaction vessel of from 0.99:1 to 0.4:1.

2. A process as claimed in claim 1, **characterized in that** it is carried out at a pressure of from 3 to 150 bar.

3. A process as claimed in claim 1, **characterized in that** it is carried out at a pressure of from 5 to 100 bar.

4. A process as claimed in any of claims 1 to 3, **characterized in that** the liquid-side volumetric mass transfer coefficient is from 0.2 s⁻¹ to 1 s⁻¹.

5. A process as claimed in any of claims 1 to 4, **characterized in that** the catalyst used comprises at least one element selected from among the elements of transition groups I, VI, VII and VIII of the Periodic Table of the Elements.

6. A process as claimed in claim 5, **characterized in that** the catalyst comprises at least one of the elements copper, chromium, molybdenum, manganese, rhenium, iron, ruthenium, cobalt, nickel, platinum and palladium.

7. A process as claimed in claim 5 or 6, **characterized in that** the catalyst comprises up to 5% by weight of at least one element selected from among the elements of main groups II, III, IV and VI, transition groups II, III, IV and V of the Periodic Table of the Elements and the lanthanides.

8. A process as claimed in any of claims 5 to 7, **characterized in that** the catalyst comprises a support selected from among the oxides of aluminum and titanium, zirconium dioxide, silicon dioxide, clays, silicates, zeolites and activated carbon.

## Revendications

1. Procédé de préparation de 1,4 butanediol par hydrogénation catalytique continue de 1,4 butinediol, **caractérisé en ce que** l'on fait réagir du 1,4 butinediol avec de l'hydrogène dans la phase continue liquide, en présence d'un catalyseur d'hydrogénation hétérogène, à des températures de 20 à 300°C, sous une pression de 1 à 200 bars et avec des valeurs de coefficient de transfert de matière rapporté au volume côté liquide hₗa, de 0,1 s⁻¹ à 1s⁻¹,
a) avec un catalyseur en suspension dans le milieu réactionnel, étant entendu que lorsqu'on utilise une colonne à bulles garnie, celle-ci est utilisée selon le mode ascendant et avec un rapport, de la quantité de gaz sortant du récipient à réaction à la quantité de gaz admise dans ce dernier, 0,99/1 à 0,4/1 ou,
b) on fait passer l'hydrogène à co-courant ascendant dans un réacteur à lit fixe fonctionnant avec recirculation du gaz, et **en ce que** l'on maintient un rapport de la quantité de gaz sortant du récipient à réaction à la quantité de gaz admise dans ce dernier de 0,99/1 à 0,4/1.

2. Procédé selon la revendication 1, **caractérisé en ce qu'**il est mis en oeuvre sous une pression de 3 à 150 bars.

3. Procédé selon la revendication 1, **caractérisé en ce qu'**il est mis en oeuvre à une pression de 5 à 100 bars.

4. Procédé selon une quelconque des revendications 1 à 3, **caractérisé en ce que** le coefficient de transfert de matière rapporté au volume, côté liquide, est de 0,2 s⁻¹ à 1 s⁻¹.

5. Procédé selon une quelconque des revendications 1 à 4, **caractérisé en ce que** l'on utilise un catalyseur qui contient au moins un élément choisi parmi les éléments du groupe I., VI., VII. ou VIII du système périodique des éléments.

6. Procédé selon la revendication 5, **caractérisé en ce que** le catalyseur contient au moins un des éléments cuivre, chrome, molybdène, manganèse, rhénium, fer, ruthénium, cobalt, nickel, platine et palladium.

7. Procédé selon l'une ou l'autre des revendications 5 ou 6, **caractérisé en ce que** le catalyseur contient jusqu'à 5% en poids d'au moins un élément choisi parmi les éléments du groupe principal II., III., IV., et VI, du groupe annexe II., III., IV. et V du système périodique des éléments et les lanthanoïdes

8. Procédé selon l'une ou l'autre des revendications 5 à 7, **caractérisé en ce que** le catalyseur comporte un support choisi parmi les oxydes d'aluminium et de titane, le dioxyde de zirconium, le dioxyde de silicium, les alumines, les silicates, les zéolithes et le charbon actif.
